(19) Europäisches Patentamt
European Patent Office
Office européen des brevets

(11) EP 1 040 101 B1

(12) EUROPÄISCHE PATENTSCHRIFT

(45) Veröffentlichungstag und Bekanntmachung des Hinweises auf die Patenterteilung:
**25.04.2007 Patentblatt 2007/17**

(21) Anmeldenummer: **98966301.8**

(22) Anmeldetag: **11.12.1998**

(51) Int Cl.:
*C07D 239/94* (2006.01) *A61K 31/505* (2006.01)

(86) Internationale Anmeldenummer:
**PCT/EP1998/008097**

(87) Internationale Veröffentlichungsnummer:
**WO 1999/032460 (01.07.1999 Gazette 1999/26)**

# (54) SUBSTITUIERTE 2-ARYL-4-AMINO-CHINAZOLINE, VERFAHREN ZU IHRER HERSTELLUNG UND IHRE VERWENDUNG ALS ARZNEIMITTEL

SUBSTITUTED 2-ARYL-4-AMINO-CHINAZOLINES, METHOD FOR THE PRODUCTION AND USE THEREOF AS MEDICAMENTS

2-ARYL-4-AMINOQUINAZOLINES SUBSTITUEES, LEUR PROCEDE DE PRODUCTION ET LEUR UTILISATION COMME MEDICAMENT

(84) Benannte Vertragsstaaten:
**AT BE CH CY DE DK ES FI FR GB GR IE IT LI LU NL PT SE**

(30) Priorität: **18.12.1997 DE 19756388**

(43) Veröffentlichungstag der Anmeldung:
**04.10.2000 Patentblatt 2000/40**

(73) Patentinhaber: **Sanofi-Aventis Deutschland GmbH**
**65929 Frankfurt am Main (DE)**

(72) Erfinder:
- **SCHINDLER, Ursula**
  **D-65812 Bad Soden (DE)**
- **SCHINDLER, Peter**
  **D-65812 Bad Soden (DE)**
- **SCHÖNAFINGER, Karl**
  **D-63755 Alzenau (DE)**
- **STROBEL, Hartmut**
  **D-65835 Liederbach (DE)**

(56) Entgegenhaltungen:
**EP-A- 0 655 456**
**DE-A- 2 338 669**
**GB-A- 1 390 015**
**US-A- 3 594 480**
**US-A- 5 436 233**

- **R. L. MCKEE ET AL.: JOURNAL OF THE AMERICAN CHEMICAL SOCIETY, Bd. 68, 1946, Seiten 1902-3, XP002097666 in der Anmeldung erwähnt**
- **HOBBS A.J. BRITISH JOURNAL OF PHARMACOLOGY Bd. 136, 2002, Seiten 637 - 640**
- **STASCH J.-P. ET AL BRITISH JOURNAL OF PHARMACOLOGY Bd. 136, 2002, Seiten 773 - 783**
- **YUNDE ZHAO ET AL BIOCHEMISTRY Bd. 39, 2000, Seiten 10848 - 10854**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 040 101 B1

Printed by Jouve, 75001 PARIS (FR)

## Beschreibung

Die vorliegende Erfindung betrifft Verbindungen der Formel I,

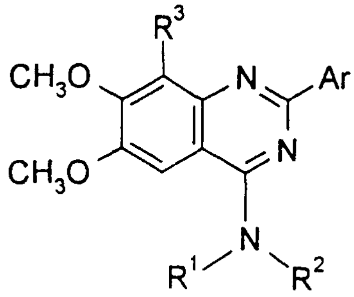

I

die sich zur Herstellung von Arzneimitteln zum Beispiel zur Prophylaxe und Therapie von Herz-Kreislauferkrankungen wie Bluthochdruck, Angina pectoris, Herzinsuffizienz, Thrombosen oder Atherosklerose eignen. Die Verbindungen der Formel I haben die Fähigkeit zur Modulation der körpereigenen Produktion von cyclischem Guanosinmonophosphat (cGMP) und eignen sich generell zur Therapie und Prophylaxe von Krankheitszuständen, die mit einem gestörten cGMP-Haushalt verbunden sind.

Cyclisches Guanosinmonophosphat (cGMP) ist ein wichtiger intrazellulärer Botenstoff, der über die Modulation cGMP-abhängiger Proteinkinasen, Phosphodiesterasen und Ionenkanäle eine Vielzahl verschiedener Effekte auslöst. Beispiele sind die Glattmuskelrelaxation, die Inhibition der Plättchenaktivierung und die Hemmung von Glattmuskelzellproliferation und Leukozytenadhäsion. cGMP wird durch partikuläre und lösliche Guanylatcyclasen als Antwort auf eine Reihe extra- und intrazellulärer Stimuli produziert. Im Falle der partikulären Guanylatcyclasen erfolgt die Stimulation im wesentlichen durch peptidische Signalstoffe, wie dem atrialen Peptid oder dem cerebralen natriuretischen Peptid. Die löslichen Guanylatcyclasen (sGC), bei denen es sich um cytosolische, heterodimere Hämproteine handelt, werden dagegen im wesentlichen durch eine Familie niedermolekularer, enzymatisch gebildeter Faktoren reguliert. Wichtigstes Stimulans ist das Stickstoffmonoxid (NO) oder eine nahe verwandte Spezies, die Bedeutung anderer Faktoren wie Kohlenmonoxid oder dem Hydroxylradikal ist noch weitgehend ungeklärt. Als Aktivierungsmechanismus wird die Anbindung von NO an das Häm unter Ausbildung eines pentakoordinierten Häm-Nitrosyl-Komplexes diskutiert. Die damit verbundene Freisetzung des im Basal-Zustand an das Eisen gebundenen Histidins, überführt das Enzym in die aktivierte Konformation. Aktive lösliche Guanylatcyctasen setzen sich aus je einer α- und einer β-Untereinheit zusammen. Von den Untereinheiten wurden mehrere Subtypen beschrieben, die sich untereinander bezüglich Sequenz, gewerbespezifischer Verteilung und Expression in verschiedenen Entwicklungsstadien unterscheiden. Die Subtypen $\alpha_1$ und $\beta_1$ werden hauptsächlich in Gehirn und Lunge exprimiert, während $\beta_2$ vor allem in Leber und Niere gefunden wird. In humanen fötalem Gehirn konnte der Subtyp $\alpha_2$ nachgewiesen werden, die als $\alpha_3$ und $\beta_3$ bezeichnetem Untereinheiten wurden aus menschlichem Gehirn isoliert und sind homolog zu $\alpha_1$ und $\beta_1$. Neuere Arbeiten weisen auf eine $\alpha_2$-Untereinheit hin, die ein Insert in der katalytischen Domäne enthält. Alle Untereinheiten zeigen große Homologien im Bereich der katalytischen Domäne. Die Enzyme enthalten vermutlich ein Häm pro Heterodimer, das über $\beta_1$-Cys-78 und/oder $\beta_1$-His-105 gebunden und Teil des regulatorischen Zentrums ist.

Unter pathologischen Bedingungen kann die Bildung Guanylatcyclaseaktivierender Faktoren vermindert sein oder durch das vermehrte Auftreten freier Radikale ein verstärkter Abbau derselben erfolgen. Die daraus resultierende, verminderte Aktivierung der sGC, führt über die Abschwächung der jeweiligen cGMP-vermittelten Zellantwort zum Anstieg des Blutdrucks, zur Plättchenaktivierung und zu vermehrter Zellproliferation und Zell-Adhäsion. Als Folge kommt es zur Ausbildung von endothelialer Dysfunktion, Atherosklerose, Bluthochdruck, stabiler und instabiler Angina pectoris, Thrombosen, Myocardinfarkt, Schlaganfällen oder erektiler Dysfunktion.

Die pharmakologische Stimulation der sGC bietet eine Möglichkeit zur Normalisierung der cGMP-Produktion und erlaubt damit die Behandlung bzw. Prävention dieser Krankheiten. Zur pharmakologischen Stimulation der sGC wurden bisher ausschließlich Verbindungen wie etwa Nitrate verwendet, deren Wirkung auf einer intermediären NO-Freisetzung beruht. Der Nachteil dieser Behandlungsweise liegt in der Toleranzentwicklung und der deshalb erforderlichen höheren Dosierung.

Verschiedene Chinazoline und pharmakologische Wirkungen von Chinazolinen sind bereits bekannt. 2-(p-Chlorphenyl)-4-(1-diethylamino-4-pentylamino)-6,7-dimethoxy-chinazolin-dihydrochlorid ist ohne Angabe der Wirkstärke im Zusammenhang mit Verbindungen, die eine Antimalaria-Wirkung aufweisen, veröffentlicht worden (R.L. McKee, M.K. McKee und R.W. Bost, J. Amer. Chem. Soc 68:1902-1903 (1946)).

2-Alkyl-chinazoline sind als bronchodilatierende und hypotensive Verbindungen beschrieben worden (US-A-3 594 480). Spezielle 2-Phenyl-chinazoline, welche Nitrato-Gruppen im 4-Amino-Substituenten enthalten, wurden als antianginöse Mittel beschrieben (DE-A-2 338 669). 2-Aryl-chinazoline, die Phosphonato-Gruppen enthalten, sind außerdem als Mittel zur Behandlung von Hyperlipidämie, Hypertension und Diabetes beschrieben worden (EP-A-0 655 456). 4-Amino-chinazolin-Derivate mit einem heterocyclischen Substituenten in der 2-Position sind als Hemmstoffe der Thromboxan $A_2$-Synthetase und/oder der cGMP-Phosphodiesterase beschrieben worden (US-A-5 436 233).

In dem Bestreben, wirksame Verbindungen zur Modulation der körpereigenen Produktion von cyclischem Guanosinmonophosphat (cGMP) zu finden, die sich zur Therapie und Prophylaxe von Krankheitszuständen eignen, die mit einem gestörten cGMP-Haushalt verbunden sind, wurde nun gefunden, daß die Chinazoline der Formel I eine starke Guanylatcyclase-Aktivierung bewirken, und damit zur Behandlung von Krankheiten geeignet sind, die mit einem niedrigen cGMP-Spiegel in Verbindung stehen.

Die vorliegende Erfindung betrifft daher Verbindungen der Formel I

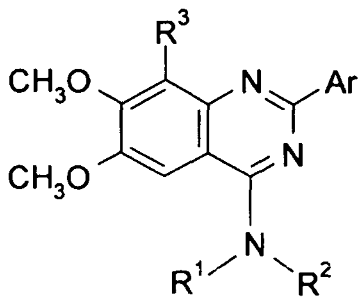

I

und/oder stereoisomere Formen der Verbindungen der Formel I und/oder Mischungen solcher Formen in allen Verhältnissen und/oder physiologisch verträgliche Salze der Verbindungen der Formel I, worin
einer der Reste $R^1$ und $R^2$ für Cyclohexyl steht, das unsubstituiert ist oder substituiert ist durch -OH,
und der andere der Reste $R^1$ und $R^2$ steht für

1. Wasserstoff,
2. $(C_1-C_5)$-Alkyl,
3. $(C_1-C_5)$-Alkyl, das einfach, zweifach oder dreifach substituiert ist durch
3.1 -OH,
3.2 -O-$(C_1-C_6)$-Alkyl,
3.3 -SH,
3.4 -$SR^4$, worin $R^4$ $(C_1-C_6)$-Alkyl bedeutet,
3.5 -$N(R^6)R^7$, worin $R^6$ und $R^7$ gleich oder verschieden sind und unabhängig voneinander Wasserstoff oder $(C_1-C_6)$-Alkyl bedeuten,
3.6 -C(O)-$N(R^6)R^7$, worin $R^6$ und $R^7$ gleich oder verschieden sind und unabhängig voneinander Wasserstoff oder $(C_1-C_6)$-Alkyl bedeuten, oder $R^6$ und $R^7$ zusammen mit dem N-Atom, an das sie gebunden sind, einen Morpholin-, Piperazin-, Imidazol-, Piperidin-, Pyrrolidin-,Thiomorpholin-, 1-Oxo-thiomorpholin-, 1,1-Dioxo-thiomorpholin- oder Hexamethylenimin-Rest bilden,
3.7 -O-$(C_1-C_6)$-Alkyl, das einfach, zweifach oder dreifach substituiert ist durch
    3.7.1 -OH,
    3.7.3 =O,
    3.7.4 -COOH,
3.8 -COOH,
3.9 -C(O)-O-$R^8$, worin $R^8$ $(C_1-C_6)$-Alkyl bedeutet,
3.10 Phenyl,
3.11 Phenyl, das einfach, zweifach oder dreifach substituiert ist durch
    3.11.1 -O-$(C_1-C_4)$-Alkyl,
    3.11.2 -O-Phenyl,
    3.11.3 $(C_1-C_4)$-Alkyl,
    3.11.4 -$NO_2$,
    3.11.5 Halogen,
    3.11.6 $C(R^9)(R^{10})R^{11}$, worin $R^9$, $R^{10}$ und $R^{11}$ unabhängig voneinander Wasserstoff oder Halogen bedeuten,
3.12 einen Rest eines Heterocyclus aus der Gruppe Morpholin, Piperazin, Imidazol, Piperidin, Pyrrolidin, Pyridin, Thiomorpholin, 1-Oxo-thiomorpholin, 1,1-Dioxo-thiomorpholin, Hexamethylenimin, Pyrrol, Pyrazol, Pyridazin,

Pyrazin, Pyrimidin, Indolizin, Indol, Indazol, Purin, Chinoxalin, Furan, Chinazolin, Cinnolin, Pteridin, Oxazol, Isoxazol, Thiazol, Isothiazol, Furazan, Indolin, Pyrazolin, Thiophen, Xanthin, Imidazolin und Pyran,

3.13 einen Rest eines in 3.12 beschriebenen Heterocyclus, der einfach, zweifach, dreifach oder vierfach substituiert ist durch

3.13.1 ($C_1$-$C_4$)-Alkyl,
3.13.2 =O,
3.13.3 Halogen,
3.13.4 O-($C_1$-$C_4$)-Alkyl,
3.13.5 $-NO_2$

4. ($C_3$-$C_7$)-Cycloalkyl, das unsubstituiert ist oder substituiert ist durch

4.1 ($C_1$-$C_4$)-Alkyl,
4.2 -OH,
4.3 -O-($C_1$-$C_4$)-Alkyl,
4.4 $-NH_2$,

$R^3$ für Wasserstoff oder Methoxy steht,

Ar für Phenyl steht, das einfach, zweifach oder dreifach substituiert ist durch

1. Halogen,
2. $-NO_2$,
3. -O-($C_1$-$C_6$)-Alkyl,
4. ($C_1$-$C_6$)-Alkyl, das unsubstituiert ist oder einfach, zweifach oder dreifach substituiert ist durch Halogen,
5. -CN,
6. -C(O)-N($R^{12}$)$R^{13}$, worin $R^{12}$ und $R^{13}$ gleich oder verschieden sind und unabhängig voneinander Wasserstoff oder ($C_1$-$C_6$)-Alkyl bedeuten.

Bevorzugt sind Verbindungen der Formel I und/oder stereoisomere Formen der Verbindungen der Formel I und/oder Mischungen solcher Formen in allen Verhältnissen und/oder physiologisch verträgliche Salze der Verbindungen der Formel I, worin
einer der Reste $R^1$ und $R^2$ für Cyclohexyl steht, das unsubstituiert ist oder substituiert ist durch -OH,
und der andere der Reste $R^1$ und $R^2$ steht für

1. Wasserstoff,
2. ($C_1$-$C_3$)-Alkyl,
3. ($C_1$-$C_3$)-Alkyl, das einfach, zweifach oder dreifach substituiert ist durch

3.1 -OH,
3.2 -O-($C_1$-$C_3$)-Alkyl,
3.3 -N($R^6$)$R^7$, worin $R^6$ und $R^7$ gleich oder verschieden sind und unabhängig voneinander Wasserstoff oder ($C_1$-$C_3$)-Alkyl bedeuten,
3.4 -C(O)-N($R^6$)$R^7$, worin $R^6$ und $R^7$ gleich oder verschieden sind und unabhängig voneinander Wasserstoff oder ($C_1$-$C_4$)-Alkyl bedeuten, oder $R^6$ und $R^7$ zusammen mit dem N-Atom, an das sie gebunden sind, einen Morpholin-, Piperazin-, Imidazol-, Piperidin-, Pyrrolidin-, Thiomorpholin-, 1-Oxo-thiomorpholin-, 1,1-Dioxo-thiomorpholin- oder Hexamethylenimin-Rest bilden,
3.5 -O-($C_1$-$C_6$)-Alkyl, das einfach substituiert ist durch -OH,
3.6 -COOH,
3.7 -C(O)-O-$R^8$, worin $R^8$ ($C_1$-$C_4$)-Alkyl bedeutet,
3.8 Phenyl,
3.9 Phenyl, das einfach, zweifach oder dreifach substituiert ist durch
3.9.1 -O-($C_1$-$C_4$)-Alkyl,
3.9.2 -O-Phenyl,
3.9.3 ($C_1$-$C_4$)-Alkyl,
3.9.4 $-NO_2$,
3.10 einen Rest eines Heterocyclus aus der Gruppe Morpholin, Piperazin, Imidazol, Piperidin, Pyrrolidin, Pyridin, Thiomorpholin, 1-Oxo-thiomorpholin, 1,1-Dioxo-thiomorpholin, Hexamethylenimin, Pyrrol, Pyrazol, Purin und Pyrimidin,
3.11 einen Rest eines in 3.10 beschriebenen Heterocyclus, der einfach, zweifach, dreifach oder vierfach substituiert ist durch
3.11.1 ($C_1$-$C_4$)-Alkyl,
3.11.2 =O,

4. ($C_5$-$C_6$)-Cycloalkyl, das unsubstituiert ist oder substituiert ist durch

4.1 $(C_1-C_4)$-Alkyl,
4.2 -OH,
4.3 $-O-(C_1-C_4)$-Alkyl,
4.4 $-NH_2$,

$R^3$ für Wasserstoff oder Methoxy steht,

Ar für Phenyl steht, das einfach, zweifach oder dreifach substituiert ist durch

1. Halogen,
2. $-NO_2$,
3. $-O-(C_1-C_3)$-Alkyl,
4. $(C_1-C_2)$-Alkyl, das unsubstituiert ist oder einfach, zweifach oder dreifach substituiert ist durch Halogen,
5. $-C(O)-NH_2$.

Insbesondere bevorzugt sind Verbindungen der Formel I und/oder stereoisomere Formen der Verbindungen der Formel I und/oder Mischungen solcher Formen in allen Verhältnissen und/oder physiologisch verträgliche Salze der Verbindungen der Formel I, worin
einer der Reste $R^1$ und $R^2$ für Cyclohexyl steht, das unsubstituiert ist oder substituiert ist durch -OH,
und der andere der Reste $R^1$ und $R^2$ steht für

1. Wasserstoff,
2. $(C_1-C_3)$-Alkyl,
3. $(C_1-C_3)$-Alkyl, das einfach oder zweifach substituiert ist durch
   3.1 -OH,
   3.2 $-O-CH_3$,
   3.3 $-N(R^6)R^7$, worin $R^6$ und $R^7$ gleich oder verschieden sind und unabhängig voneinander Wasserstoff oder $(C_1-C_3)$-Alkyl bedeuten,
   3.4 $-O-(C_1-C_2)$-Alkyl, das einfach oder zweifach substituiert ist durch -OH,
   3.5 einen Rest eines Heterocyclus aus der Gruppe Morpholin und Pyridin,
4. $(C_5-C_6)$-Cycloalkyl, das unsubstituiert ist oder substituiert ist durch
   4.1 $(C_1-C_4)$-Alkyl,
   4.2 -OH,
   4.3 $-O-(C_1-C_4)$-Alkyl,
   4.4 $-NH_2$,

$R^3$ für Wasserstoff oder Methoxy steht,

Ar für Phenyl steht, das einfach, zweifach oder dreifach substituiert ist durch

1. Halogen,
2. $-NO_2$,
3. $-O-(C_1-C_3)$-Alkyl,
4. $(C_1-C_2)$-Alkyl, das unsubstituiert ist oder einfach, zweifach oder dreifach substituiert ist durch Halogen,
5. $-C(O)-NH_2$.

In den Verbindungen der Formel I und/oder stereoisomeren Formen der Verbindungen der Formel I und/oder Mischungen solcher Formen in allen Verhältnissen und/oder physiologisch verträglichen Salzen der Verbindungen der Formel I, in denen einer der Reste $R^1$ und $R^2$ für unsubstituiertes oder durch -OH substituiertes Cyclohexyl steht, kann der andere der Reste $R^1$ und $R^2$ für Wasserstoff oder für Alkyl oder für Cycloalkyl stehen. Wenn der andere der Reste $R^1$ und $R^2$ für einen Cycloalkylrest steht, steht er bevorzugt für einen unsubstituierten Cycloalkylrest oder einen substituierten Cycloalkylrest, der einen, zwei oder drei Substituenten, insbesondere einen oder zwei Substituenten, trägt. Bevorzugte Substituenten in derartigen substituierten Cycloalkylresten sind Alkylgruppen und Hydroxygruppen, insbesondere Hydroxygruppen. Ganz besonders bevorzugt sind als substituierte Cycloalkylreste solche Cycloalkylreste, die eine Hydroxygruppe als Substituenten tragen. Cycloalkylreste in derartigen Verbindungen der Formel 1 sind bevorzugt Cyclopentylreste und Cyclohexylreste. Speziell bevorzugte substituierte Cycloalkylreste sind in derartigen Verbindungen Hydroxycyclopentylreste und Hydroxycyclohexylreste. Ein Beispiel für einen durch -OH substituierten Cyclohexylrest, der für den einen der beiden Reste $R^1$ und $R^2$ stehen kann, und für einen Hydroxycyclohexylrest, der für den anderen der Reste $R^1$ und $R^2$ stehen kann, ist der 4-Hydroxycyclohexylrest. Substituenten in einem durch -OH substituierten Cyclohexylrest, der für den einen der Reste $R^1$ und $R^2$ steht, und in einem substituierten Cycloalkylrest, der für den anderen der Reste $R^1$ und $R^2$ steht, können sich aber in beliebigen Positionen befinden und können in beliebiger stereochemischer Anordnung vorliegen und unabhängig voneinander cis-ständig oder trans-ständig sein.

Eine spezifische Gruppe von erfindungsgemäßen Verbindungen bilden Verbindungen der Formel I und/oder stereoisomere Formen der Verbindungen der Formel I und/oder physiologisch verträgliche Salze der Verbindungen der

Formel I, wobei
einer der Reste $R^1$ und $R^2$ für Cyclohexyl steht,
und der andere der Reste $R^1$ und $R^2$ steht für

1. ein Wasserstoffatom,

2. $(C_1-C_5)$-Alkyl, 3. $(C_1-C_5)$-Alkyl, das einfach, zweifach oder dreifach substituiert ist durch
   3.1 -OH,
   3.2. -O-$(C_1-C_6)$-Alkyl,
   3.3 -SH,
   3.5 -$SR^4$, worin $R^4$ $(C_1-C_6)$-Alkyl bedeutet,
   3.6 -$NH_2$,
   3.7 -$N(R^6)R^7$, worin $R^6$ und $R^7$ gleich oder verschieden sind und unabhängig voneinander ein Wasserstoffatom oder $(C_1-C_6)$-Alkyl bedeuten,
   3.8 -C(O)-$NH_2$
   3.9 -C(O)-$N(R^6)R^7$, worin $R^6$ und $R^7$ gleich oder verschieden sind und unabhängig voneinander ein Wasserstoffatom oder $(C_1-C_6)$-Alkyl bedeuten, oder $R^6$ und $R^7$ zusammen mit dem N-Atom, an das sie gebunden sind einen Morpholin-, Piperazin-, Imidazol-, Piperidin-, Pyrrolidin-, Thiomorpholin-, 1-Oxo-thiomorpholin-, 1,1-Dioxo-thiomorpholin- oder einen Hexamethylenimin-Rest bilden,
   3.10 -O-$(C_1-C_6)$-Alkyl, das einfach, zweifach oder dreifach substituiert ist durch
   3.10.1 -OH,
   3.10.2 -SH,
   3.10.3 =O oder
   3.10.4 -COOH,
   3.11 -COOH,
   3.12 -C(O)-O-$R^8$, worin $R^8$ $(C_1-C_6)$-Alkyl bedeutet,
   3.13 Phenyl,
   3.14 Phenyl, worin der Phenylring einfach, zweifach oder dreifach substituiert ist durch
   3.14.1 -O-$(C_1-C_4)$-Alkyl,
   3.14.2 -O-Phenyl,
   3.14.3 $(C_1-C_4)$-Alkyl,
   3.14.4 -$NO_2$,
   3.14.5 Halogen oder
   3.14.6 -$C(R^9)(R^{10})R^{11}$, worin $R^9$, $R^{10}$ und $R^{11}$ unabhängig voneinander ein Wasserstoffatom oder Halogen bedeuten,
   3.15 einen Rest eines Heterocyclus aus der Gruppe Morpholin, Piperazin, Imidazol, Piperidin, Pyrrolidin, Pyridin, Thiomorpholin, 1-Oxo-thiomorpholin, 1,1-Dioxo-thiomorpholin, Hexamethylenimin, Pyrrol, Pyrazol, Pyridazin, Pyrazin, Pyrimidin, Indolizin, Indol, Indazol, Purin, Chinoxalin, Furan, Chinazolin, Cinnolin, Pteridin, Oxazol, Isoxazol, Thiazol, Isothiazol, Furazan, Indolin, Pyrazolin, Thiophen, Xanthin, Imidazolin oder Pyran, oder
   3.16 einen Rest eines Heterocyclus wie in 3.15 beschrieben, der einfach, zweifach, dreifach oder vierfach substituiert ist durch
   3.16.1 $(C_1-C_4)$-Alkyl,
   3.16.2 =O,
   3.16.3 Halogen,
   3.16.4 -O-$(C_1-C_4)$-Alkyl oder
   3.16.5 -$NO_2$,

4. $(C_3-C_7)$-Cycloalkyl,

$R^3$ für ein Wasserstoffatom oder Methoxy steht und

Ar für Phenyl steht, das einfach, zweifach oder dreifach substituiert ist durch
   1. Halogen,
   2. -$NO_2$,
   3. -O-$(C_1-C_6)$-Alkyl,
   4. -$(C_1-C_6)$-Alkyl, das unsubstituiert ist oder einfach, zweifach oder dreifach substituiert ist durch Halogen,
   5. -C(O)-$NH_2$ oder
   6. -C(O)-$N(R^{12})R^{13}$, worin $R^{12}$ und $R^{13}$ gleich oder verschieden sind und unabhängig voneinander ein Wasser-

stoffatom oder ($C_1$-$C_6$)-Alkyl bedeuten.

Beispiele für substituierte Phenylreste, die für Ar stehen können, sind Halogenphenylreste, zum Beispiel Chlorphenylreste wie 3-Chlorphenyl oder 4-Chlorphenyl, Alkylphenylreste, zum Beispiel Methylphenylreste wie 3-Methylphenyl oder 4-Methylphenyl, oder Trifluormethylphenylreste, zum Beispiel 4-Trifluormethylphenyl oder 3,5-Bistrifluormethylphenyl. Eine Untergruppe von Verbindungen der Formel I wird von solchen Verbindungen gebildet, in denen der für Ar stehende Phenylrest nur einen Substituenten trägt, zum Beispiel einen Substituenten, der aus der Gruppe Halogen und ($C_1$-$C_4$)-Alkyl ausgewählt ist. Eine zweite Untergruppe wird von solchen Verbindungen der Formel I gebildet, in denen der für Ar stehende Phenylrest zwei oder drei gleiche oder verschiedene Substituenten trägt. Eine weitere Untergruppe wird von solchen Verbindungen gebildet, in denen in den Resten $R^1$ und/oder $R^2$ eine Hydroxygruppe enthalten ist. Ein Beispiel für die Gruppe -N($R^6$)$R^7$ ist die Gruppe- $NH_2$, ein Beispiel für die Gruppen -C(O)-N($R^6$)$R^7$ und -C(O)-N($R^{12}$) $R^{13}$ ist die Gruppe -C(O)-$NH_2$.

Wenn Gruppen in Verbindungen der Formel I durch mehrere Substituenten substituiert sein können, können in allen Fällen die Substituenten alle gleich sein oder teilweise gleich sein oder alle verschieden sein. Dies gilt für die Substituenten, die spezifisch als mögliche Substituenten einer Gruppe in der Definition der Verbindungen der Formel I aufgeführt sind, und gilt auch, wenn mehrere Substituenten der gleichen Art in einer Gruppe vorhanden sind, beispielsweise mehrere Halogenatome und/oder mehrere ($C_1$-$C_4$)-Alkylreste, welch letztere dann zum Beispiel Methylgruppen und/oder Ethylgruppen und/oder Butylgruppen sein können. Soweit die resultierende Gruppe bzw. das Molekül der Formel I stabil ist und aufgrund der betreffenden Substitution keine unerwünschten Eigenschaften aufweist, können Substituenten in beliebigen Kombinationen auftreten und können sich in beliebigen Positionen in einer Gruppe befinden. Erfindungsgemäße Verbindungen der Formel I enthalten im allgemeinen aber nicht mehr als zwei Nitrogruppen im Molekül.

Alkylreste können geradkettig oder verzweigt sein. Dies gilt auch, wenn sie substituiert sind, zum Beispiel durch einen Phenylrest oder durch Hydroxy, oder wenn sie in anderen Gruppen enthalten sind, zum Beispiel in Alkoxygruppen. Beispiele für Alkylgruppen sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, n-Heptyl, n-Octyl. Unter dem Begriff Alkyl sind hier auch ungesättigte Alkylreste zu verstehen, insbesondere Alkylreste, die eine oder zwei Doppelbindungen oder eine oder zwei Dreifachbindungen oder eine Doppelbindung und eine Dreifachbindung enthalten. Beispiele für solche Reste sind der Vinylrest, der 2-Propenylrest (Allylrest), der 2-Butenylrest, der 3-Methyl-2-butenylrest, der Ethinylrest, der 2-Propinylrest (Propargylrest) oder der 3-Butinylrest.

Beispiele für Cycloalkyl sind Cyclopropyl, Cyclobutyl, Cyclopentyl, Cyclohexyl oder Cycloheptyl. Substituierte Cycloalkylreste sind bevorzugt durch einen, zwei, drei oder vier gleiche oder verschiedene Substituenten substituiert, besonders bevorzugt durch einen oder zwei gleiche oder verschiedene Substituenten.

Halogen bedeutet Fluor, Chlor, Brom oder Iod, bevorzugt Fluor oder Chlor.

In monosubstituierten Phenylresten kann sich der Substituent in der 2-Position, der 3-Position oder der 4-Position befinden. Ist Phenyl zweifach substituiert, können die Substituenten sich in der 2,3-Position, der 2,4-Position, der 2,5-Position, der 2,6-Position, der 3,4-Position oder der 3,5-Position befinden. In dreifach substituierten Phenylresten können sich die Substituenten in der 2,3,4-Position, der 2,3,5-Position, der 2,4,5-Position, der 2,4,6-Position, der 2,3,6-Position oder der 3,4,5-Position befinden.

Heterocyclische Reste können über alle geeigneten Atome gebunden sein, sowohl über Kohlenstoffatome als auch über Stickstoffatome, soweit dies in Einklang mit der jeweiligen Definition des Substituenten ist. Beispielsweise kann ein Piperidinrest ein 1-Piperidinylrest (= Piperidinorest), ein 2-Piperidinylrest, ein 3-Piperidinylrest oder ein 4-Piperidinylrest sein. Imidazolyl kann 1-Imidazolyl, 2-Imidazolyl, 4-Imidazolyl oder 5-Imidazolyl sein, Pyridyl kann 2-Pyridyl, 3-Pyridyl oder 4-Pyridyl sein.

Die Verbindungen der Formel I können bei entsprechender Substitution in stereoisomeren Formen oder in Gemischen stereoisomerer Formen vorliegen. Enthalten die Verbindungen der Formel 1 ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Mischungen dieser Formen in allen Verhältnissen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe einer Ausgangssubstanz oder eines Zwischenprodukts im Verlaufe der

Synthese. Bei Vorliegen von tautomeren Formen umfaßt die Erfindung auch alle möglichen Tautomeren.

Enthalten die Verbindungen der Formel 1 eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die eine oder mehrere saure Gruppen enthalten, zum Beispiel COOH-Gruppen in Phenylringen oder saure Hydroxygruppen, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren. Verbindungen der Formel I, die eine oder mehrere basische, das heißt protonierbare, Gruppen enthalten, können in Form ihrer Säureadditionssalze mit anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine zu der Erfindung. Salze können aus den Verbindungen der Formel 1 nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel 1, zum Beispiel Hydrate oder Addukte mit Alkoholen, sowie Derivate der Verbindungen der Formel 1, zum Beispiel Ester, Prodrugs und aktive Metabolite.

Die Erfindung betrifft ferner Verfahren zur Herstellung der Verbindungen der Formel I. Ausgangsmaterial für ein Verfahren zur Herstellung von Verbindungen der Formel I sind o-Amino-benzoesäureamide oder o-Aminobenzoesäureester der Formel II, in der X zum Beispiel für Amino oder -O-($C_1$-$C_4$)-Alkyl stehen kann. Die Verbindungen der Formel II können mit Benzoesäuren oder deren aktivierten Derivaten, zum Beispiel den Benzoesäurechloriden der Formel III, zu den Verbindungen der Formel IV umgesetzt werden. Die Verbindungen der Formel IV können dann zu den 4-Hydroxy-chinazolinen der Formel V umgesetzt werden, die zum Beispiel durch Chlorierung mit Chlorierungsmitteln zu Verbindungen der Formel VI umgesetzt werden können. Aus den Verbindungen der Formel VI und den gewünschten Aminen der Formel HN($R^1$)$R^2$ können anschließend durch Austausch des Chloratoms gegen die Aminogruppe die Verbindungen der Formel I erhalten werden. Geeignete Lösungsmittel für diese Austauschreaktion sind zum Beispiel Wasser, Alkohole, Tetrahydrofuran (THF), Dioxan, Dimethylformamid (DMF), N-Methylpyrrolidon (NMP), Benzol, Toluol, Xylol, Chlorbenzol, Dichlorbenzol.

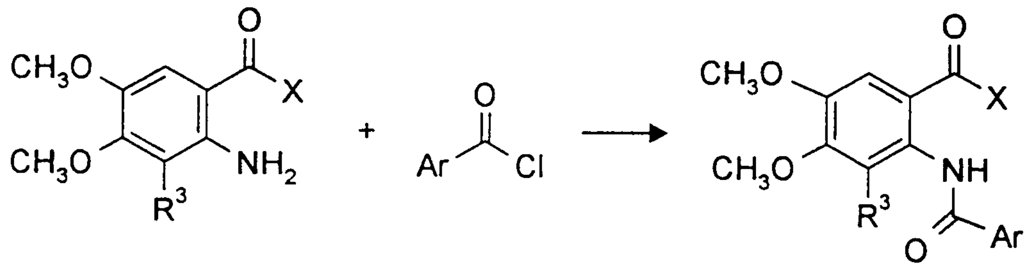

II III IV

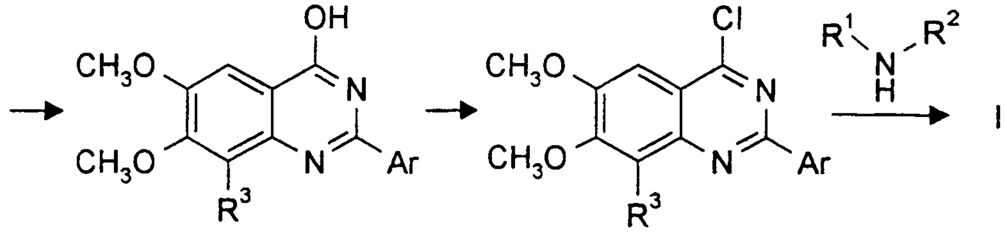

V VI

Die Chlorierung der Verbindungen der Formel V zu den Verbindungen der Formel VI kann zum Beispiel vorteilhaft mit Phosphorchloriden wie Phosphoroxychlorid und/oder Phosphorpentachlorid oder mit anderen Chlorierungsmitteln durchgeführt werden. Die Cyclisierung zu den Verbindungen der Formel V kann durch Säuren und besonders vorteilhaft mit Basen bewerkstelligt werden. Im Falle von Verbindungen der Formeln II und IV, in denen X für OAlkyl steht, wird für die Cyclisierung Ammoniak benötigt. Die Umsetzung kann dann in vorteilhafter Weise unter erhöhtem Druck erfolgen. Die Acylierung der Aminoverbindungen der Formel II mit den Arylcarbonsäurederivaten wie den Säurechloriden der Formel III kann nach bekannten Varianten der Amidherstellung erfolgen.

Diese Umsetzungen können in einem weiten Temperaturbereich durchgeführt werden. Bevorzugt sind Reaktionstemperaturen von 20 bis 150°C. Die Umsetzungen können im ersten, zweiten und letzten Schritt durch Basen wie Natriumbicarbonat, Soda, Pottasche, Triethylamin, Natriumalkoholate oder Pyridin und im letzten Schritt zusätzlich durch überschüssiges Amin beschleunigt werden. Die Zwischenstufen und die Endverbindungen der Formel I können aus den Reaktionsmischungen nach gängigen Verfahren wie Kristallisation, Sublimation oder Chromatographie, zum Beispiel Säulenchromatographie, abgetrennt und gereinigt werden. Die Ausgangsverbindungen der Formeln 11 und III sowie die Amine der Formel $R^1$ ($R^2$)NH sind kommerziell erhältlich oder in der Literatur beschrieben oder können nach bekannten Standardreaktionen hergestellt werden.

Verbindungen der Formel I, die eine Thiomorpholinogruppe enthalten, können nach bekannten Methoden, beispielsweise mit Wasserstoffperoxid in Eisessig, zu den entsprechenden Sulfoxiden und Sulfonen oxidiert werden.

Je nach den funktionellen Gruppen, die in den Ausgangsverbindungen für die Synthese der Verbindungen der Formel I enthalten sind oder die in den Endverbindungen der Formel I enthalten sein sollen, und je nach den angewandten Syntheseverfahren kann es angebracht sein, zur Vermeidung von unerwünschten Reaktionen oder Nebenreaktionen bei bestimmten Syntheseschritten Schutzgruppentechniken anzuwenden. Anstatt funktionelle Gruppen vorübergehend durch geeignete Schutzgruppen zu blockieren, können sie aber zunächst auch in Form von Vorstufen vorliegen, die dann später in die gewünschte Gruppe umgewandelt werden, zum Beispiel Aminogruppen in Form von Nitrogruppen oder Cyangruppen.

Die erfindungsgemäßen Verbindungen der Formel I bewirken über die Aktivierung der löslichen Guanylatcyclase (sGC) eine Erhöhung der cGMP-Konzentration und sind deshalb wertvolle Agenzien zur Therapie und Prophylaxe von Krankheiten, die mit einem niedrigen oder erniedrigten cGMP-Spiegel verbunden sind oder durch einen solchen verursacht werden oder zu deren Therapie oder Prophylaxe eine Erhöhung des vorhandenen cGMP-Spiegels angestrebt wird. Die Aktivierung der sGC durch die Verbindungen der Formel I kann zum Beispiel in dem unten beschriebenen Aktivitäts-Assay untersucht werden.

Krankheiten und pathologische Zustände, die mit einem niedrigen cGMP-Spiegel verbunden sind oder bei denen eine Erhöhung des cGMP-Spiegels angestrebt wird und zu deren Therapie und Prophylaxe Verbindungen der

Formel I eingesetzt werden können, sind zum Beispiel Herz-KreislaufErkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, oder zum Beispiel erektile Dysfunktion, Asthma bronchiale, chronische Niereninsuffizienz und Diabetes. Verbindungen der Formel I können darüber hinaus eingesetzt werden bei der Therapie der Leberzirrhose sowie zur Verbesserung einer eingeschränkten Lernfähigkeit oder Gedächtnisleistung.

Die Verbindungen der Formel I und ihre physiologisch verträglichen Salze sowie andere physiologisch verträgliche Derivate, zum Beispiel Prodrugs, können somit am Tier, bevorzugt am Säugetier, und insbesondere am Menschen als Arzneimittel für sich allein, in Mischungen untereinander oder in Form von pharmazeutischen Präparaten verwendet werden. Gegenstand der vorliegenden Erfindung sind daher auch die Verbindungen der Formel I und ihre physiologisch verträglichen Salze und Derivate zur Anwendung als Arzneimittel, ihre Verwendung zur Normalisierung eines gestörten cGMP-Haushalts und insbesondere ihre Verwendung in der Therapie und Prophylaxe der oben genannten Krankheitsbilder, sowie ihre Verwendung zur Herstellung von Medikamenten dafür.

Die vorliegende Erfindung betrifft auch Arzneimittel und pharmazeutische Präparate, die dadurch gekennzeichnet sind, das sie einen wirksamen Gehalt an mindestens einer Verbindung der Formel I und/oder eines physiologisch verträglichen Salzes davon und/oder eines anderen physiologisch verträglichen Derivats davon, zum Beispiel eines Prodrugs, zusammen mit einem pharmazeutisch geeigneten und physiologisch verträglichen Träger enthalten. Die pharmazeutischen Präparate enthalten normalerweise 0.2 bis 500 mg, vorzugsweise 1 bis 200 mg Wirkstoff der Formel 1 und/oder dessen physiologisch verträgliche Salze und/oder Derivate pro Dosis.

Die Arzneimittel können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen; oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die pharmazeutischen Präparate enthalten normalerweise 0.5 bis 90 Gewichtsprozent der Verbindungen der Formel I und/oder ihrer physiologisch verträglichen Salze und/oder Derivate. Die Herstellung der pharmazeutischen Präparate kann in an sich bekannter Weise erfolgen. Dazu werden ein oder mehrere Verbindungen der Formel und/oder ihre physiologisch verträglichen Salze und/oder Derivate zusammen mit einem oder mehreren festen oder flüssigen galenischen Trägerstoffen und/oder Hilfsstoffen und, wenn gewünscht, in Kombination mit anderen Arzneimittelwirkstoffen mit therapeutischer oder prophylaktischer Wirkung in eine geeignete Verabreichungsform oder Dosierungsform gebracht, die dann als Arzneimittel in der Humanmedizin oder Veterinärmedizin verwendet werden kann.

Für die Herstellung beispielsweise von Pillen, Tabletten, Dragees und Hartgelatinekapseln kann man Lactose, Stärke, zum Beispiel Maisstärke, oder Stärkederivate, Talk, Stearinsäure oder deren Salze, verwenden. Trägerstoffe für Weichgelatinekapseln und Suppositorien sind zum Beispiel Fette, Wachse, halbfeste und flüssige Polyole, natürliche oder gehärtete Öle. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle. Die Verbindungen der Formel I und ihre physiologisch verträglichen Salze können auch lyophilisiert werden und die erhaltenen Lyophilisate zum Beispiel zur Herstellung von Injektions- oder Infusionspräparaten verwendet werden. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch übliche Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, Salze zur Veränderung des osmotischen Drucks, Überzugsmittel oder Antioxidantien. Sie können weiterhin einen oder mehrere andere Arzneimittelwirkstoffe enthalten.

Die Dosierung des zu verabreichenden Wirkstoffs der Formel I und/oder eines physiologisch verträglichen Salzes und/oder Derivates davon hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel I weitere Wirkstoffe verabreicht werden. Im allgemeinen ist eine Tagesdosis von etwa 0.01 bis 100 mg/kg, vorzugsweise 0.1 bis 10 mg/kg, insbesondere 0.3 bis 5 mg/kg (jeweils mg pro kg Körpergewicht) bei Verabreichung an einen ca. 75 kg schweren Erwachsenen zur Erzielung wirksamer Ergebnisse angemessen. Die Tagesdosis kann in einer Einzeldosis verabreicht werden oder, insbesondere bei der

Applikation größerer Mengen, in mehrere, zum Beispiel zwei, drei oder vier Einzeldosen aufgeteilt werden. Gegebenenfalls kann es, je nach individuellem Verhalten, erforderlich werden, von der angegebenen Tagesdosis nach oben oder nach unten abzuweichen.

Die Verbindungen der Formel I aktivieren die lösliche Guanylatcyclase. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Beeinflussung der Guanylatcyclase beabsichtigt ist, sowie für diagnostische Zwecke, zum Beispiel in der in vitro-Diagnostik von Zellproben oder Gewebsproben. Ferner können die Verbindungen der Formel I und ihre Salze als Zwischenprodukte zur Herstellung weiterer Arzneimittelwirkstoffe dienen, die zum Beispiel aus den Verbindungen der Formel I durch Abwandlungen von funktionellen Gruppen oder Einführung von Substituenten erhältlich sind.

Die nachfolgenden Beispiele erläutern die Erfindung.

Beispiel 1

2-(4-Chlorbenzoyl-amino)-3,4,5-trimethoxy-benzoesäure-methylester (Zwischenprodukt)

Zur Lösung von 25.3 g 2-Amino-3,4,5-trimethoxy-benzoesäure-methylester und 16 ml Triethylamin in 100 ml THF wurde eine Lösung von 20.3 g 4-Chlorbenzoylchlorid in 60 ml THF getropft. Die Mischung wurde 1 Stunde (h) am Rückfluß gekocht, im Eisbad abgekühlt und filtriert. Nach dem Einengen verblieb ein farbloser Rückstand. Ausbeute: 34.1 g. Schmelzpunkt (Fp.): 116°C.

Beispiel 2

2-(4-Chlorphenyl)-4-hydroxy-6,7,8-trimethoxy-chinazolin (Zwischenprodukt)

Eine Suspension von 28.5 g 2-(4-Chlorbenzoyl-amino)-3,4,5-trimethoxy-benzoesäure-methylester in 200 ml Methanol wurde mit 150 ml flüssigem Ammoniak versetzt und 5 h im Autoklaven auf 100°C erhitzt. Beim Erkalten fiel ein Niederschlag aus, der abgesaugt und unter verminderten Druck getrocknet wurde. Ausbeute: 25 g. Fp.: 289°C.

Beispiel 3

2-(4-Chlorphenyl)-4-chlor-6,7,8-trimethoxy-chinazolin (Zwischenprodukt)

18.2 g 2-(4-Chlorphenyl)-4-hydroxy-6,7,8-trimethoxy-chinazolin wurden in 120 ml Phosphoroxychlorid 3 h auf 100°C erhitzt. Das überschüssige Phosphoroxychlorid wurde abdestilliert und der ölige Rückstand mit Eiswasser verrührt. Der Feststoff wurde abgesaugt und unter verminderten Druck getrocknet. Ausbeute: 15.0 g. Fp.: 159°C.

Beispiel 4

2-Nitro-4,5-dimethoxy-benzoesäure-amid (Zwischenprodukt)

Eine Mischung aus 45.5 g 2-Nitro-4,5-dimethoxy-benzoesäure und 120 ml Thionylchlorid wurde auf 80°C erhitzt, bis eine klare Lösung entstanden war. Das überschüssige Thionylchlorid wurde abdestilliert, der Rückstand mit Toluol versetzt und erneut eingeengt. Der ölige Rückstand wurde zu 300 ml konzentrierter wäßriger Ammoniaklösung getropft. Nach kurzem Verrühren wurde der Niederschlag abgesaugt und unter verminderten Druck getrocknet. Ausbeute: 29 g. Fp.: 201 °C.

Beispiel 5

2-Amino-4,5-dimethoxy-benzoesäure-amid (Zwischenprodukt)

Eine Suspension von 28 g 2-Nitro-4,5-dimethoxy-benzoesäure-amid wurde in Gegenwart von 1.5 g Platindioxidhydrat unter Normaldruck hydriert, bis kein Wasserstoff mehr aufgenommen wurde. Es wurde vom Katalysator abgesaugt, das Filtrat eindampft und der Rückstand unter verminderten Druck getrocknet. Ausbeute: 24.1 g. Fp.: 147°C.

Beispiel 6

2-(4-Chlorbenzoyl-amino)-4,5-dimethoxy-benzoesäure-amid (Zwischenprodukt)

Eine Mischung aus 13.7 g 2-Amino-4,5-dimethoxy-benzoesäure-amid, 8.1 g Triethylamin, 13.8 g 4-Chlorbenzoylchlorid und 300 ml Methylenchlorid wurde 2 h ohne Kühlung gerührt. Der Niederschlag wurde abgesaugt, mit Wasser verrührt, abgesaugt und unter verminderten Druck getrocknet. Ausbeute: 22.5 g. Fp.: 243°C.

Beispiel 7

2-(4-Chlorphenyl)-4-hydroxy-6,7-dimethoxy-chinazolin (Zwischenprodukt)

21.3 g 2-(4-Chlorbenzoyl-amino)-4,5-dimethoxy-benzoesäure-amid wurden in 250 ml 10 %iger Natronlauge 2 h auf 100°C erhitzt. Die Ausgangsverbindung ging dabei allmählich in Lösung und wenig später fiel erneut ein Niederschlag aus. Die Mischung wurde mit 500 ml Wasser verdünnt und mit konzentrierter Salzsäure auf pH = 4 eingestellt. Nach 2 h Nachrühren wurde der Feststoff abgesaugt, mit viel Wasser gewaschen und unter verminderten Druck bei 40°C getrocknet. Ausbeute: 19.0 g. Fp.: 329°C.

Beispiel 8

2-(4-Chlorphenyl)-4-chlor-6,7-dimethoxy-chinazolin (Zwischenprodukt)

Die Herstellung erfolgte analog Beispiel 3. Fp.: 290°C.

Beispiel 9

2-(4-Chlorphenyl)-4-(4-benzyl-piperazino)-6,7,8-trimethoxy-chinazolin (ReferenzBeispiel für die Herstellung der 4-Amino-chinazoline)

Eine Mischung aus 2.0 g 2-(4-Chlorphenyl)-4-chlor-6,7,8-trimethoxy-chinazolin und 5.0 g N-Benzylpiperazin wurde 1 h auf 150°C erhitzt. Nach dem Erkalten wurden 20 ml Eiswasser zugefügt und es wurde mit Essigester extrahiert. Die organische Phase wurde über Natriumsulfat getrocknet und eingeengt. Das so erhaltene ölige Rohprodukt wurde aus Isopropanol umkristallisiert. Ausbeute: 1 g. Fp.: 150°C.

In analoger Weise wurden die folgenden Verbindungen hergestellt.

Beispiel 15

2-(4-Trifluormethyl-phenyl)-4-hydroxy-6,7-dimethoxy-chinazolin (Zwischenprodukt)

Fp.: 338°C

Beispiel 16

2-(4-Trifluormethyl-phenyl)-4-chlor-6,7-dimethoxy-chinazolin (Zwischenprodukt)

Fp.: 181°C

Beispiel 29

2-(4-Methylphenyl)-4-hydroxy-6,7-dimethoxy-chinazolin (Zwischenprodukt)

Fp.: 305°C

Beispiel 31

2-(4-Methylphenyl)-4-chlor-6,7-dimethoxy-chinazolin (Zwischenprodukt)

Fp.: 289°C

Beispiel 60

2-(3,5-Bis-trifluormethyl-phenyl)-4-hydroxy-6,7,8-trimethoxy-chinazolin (Zwischenprodukt)

Fp.: 335°C

Beispiel 61

2-(3,5-Bis-trifluormethyl-phenyl)-4-chlor-6,7,8-trimethoxy-chinazolin (Zwischenprodukt)

Fp.: 163°C

Beispiel 93

2-(4-Chlorphenyl)-4-(trans-4-hydroxy-cyclohexylamino)-6,7-dimethoxychinazolin

Fp.: 261 °C

Beispiel 94

2-(4-Chlorphenyl)-4-(trans-4-hydroxy-cyclohexylamino)-6,7,8-trirnethoxy-chinazolin

Fp.: 199°C

Beispiel 96

2-(4-Methylphenyl)-4-(trans-4-hydroxy-cyclohexylamino)-6,7-dimethoxychinazolin

Fp.: 243°C

Pharmakologische Untersuchung

Aktivierung der löslichen Guanylatcyclase

Die Aktivierung der löslichen Guanylatcyclase (sGC), die die Umwandlung von Guanosintriphosphat (GTP) in cyclisches Guanosinmonophosphat (cGMP) und Pyrophosphat katalysiert, durch die erfindungsgemäßen Verbindungen wurde mit Hilfe eines Enyzm-Immuno-Assays (EIA) der Firma Amersham quantifiziert. Dazu wurden die Prüfsubstanzen zunächst mit sGC in Mikrotiterplatten inkubiert und dann die Menge des entstandenen cGMP bestimmt. Die eingesetzte sGC war aus Rinderlunge isoliert worden (siehe Methods in Enzymology, Band 195, S. 377). Die Testlösungen (100 µl pro well) enthielten 50 mM Triethanolamin(TEA)-Puffer (pH 7.5), 3 mM $MgCl_2$, 3 mM reduziertes Glutathion (GSH), 0.1 mM GTP, 1 mM 3-Isobutyl-1-methylxanthin (IBMX), geeignet verdünnte Enzymlösung sowie die Prüfsubstanz bzw. bei den Kontrollversuchen Lösungsmittel. Die Prüfsubstanzen wurden in Dimethylsulfoxid (DMSO) gelöst und die Lösung mit DMSO/Wasser verdünnt, so daß die Endkonzentration an Prüfsubstanz in der Testlösung 50 µM betrug. Die DMSO-Konzentration in der Testlösung betrug 5 % (v/v). Die Reaktion wurde durch Zugabe der sGC gestartet. Der Reaktionsmix wurde für 15 bis 20 Minuten bei 37°C inkubiert und dann durch Eiskühlung und Zugabe des Stop-Reagenz (50 mM EDTA, pH 8.0) gestoppt. Ein Aliquot von 50 µl wurde entnommen und zur Bestimmung des cGMP-Gehaltes mit dem Acetylierungs-Protokoll des Amersham-cGMP-EIA-Kits eingesetzt. Die Absorption der Proben wurde bei 450 nm (Referenz-Wellenlänge 620 nm) in einem Mikrotiterplatten-Lesegerät gemessen. Die cGMP-Konzentration wurde über eine Eichkurve ermittelt, die unter denselben Versuchsbedingungen erhalten wurde. Die Aktivierung der sGC durch eine Prüfsubstanz wird angegeben als n-fache Stimulation der basalen Enzymaktivität, die bei den Kontrollversuchen (mit Lösungsmittel statt Prüfsubstanz) gefunden wurde (berechnet nach der Formel

$$\text{n-fache Stimulierung} = [\text{cGMP}]_{\text{Prüfsubstanz}} / [\text{cGMP}]_{\text{Kontrolle}}).$$

Es wurden die folgenden Ergebnisse erhalten.

| Beispiel | n-fache Stimulierung | Konzentration (μM) |
|---|---|---|
| 93 | 14 | 50 |
| 94 | 13 | 50 |
| 96 | 16 | 50 |

**Patentansprüche**

**1.** Verbindung der Formel I

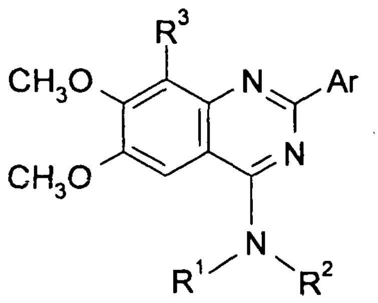

I

und/oder stereoisomere Formen der Verbindung der Formel I und/oder Mischungen solcher Formen in allen Verhältnissen und/oder physiologisch verträgliche Salze der Verbindung der Formel 1, worin
einer der Reste $R^1$ und $R^2$ für Cyclohexyl steht, das unsubstituiert ist oder substituiert ist durch -OH,
und der andere der Reste $R^1$ und $R^2$ steht für

1. Wasserstoff,
2. ($C_1$-$C_5$)-Alkyl,
3. ($C_1$-$C_5$)-Alkyl, das einfach, zweifach oder dreifach substituiert ist durch

3.1 -OH,
3.2 -O-($C_1$-$C_6$)-Alkyl,
3.3 -SH,
3.4 -$SR^4$, worin $R^4$ ($C_1$-$C_6$)-Alkyl bedeutet,
3.5 -$N(R^6)R^7$, worin $R^6$ und $R^7$ gleich oder verschieden sind und unabhängig voneinander Wasserstoff oder ($C_1$-$C_6$)-Alkyl bedeuten,
3.6 -C(O)-$N(R^6)R^7$, worin $R^6$ und $R^7$ gleich oder verschieden sind und unabhängig voneinander Wasserstoff oder ($C_1$-$C_6$)-Alkyl bedeuten, oder $R^6$ und $R^7$ zusammen mit dem N-Atom, an das sie gebunden sind, einen Morpholin-, Piperazin-, Imidazol-, Piperidin-, Pyrrolidin-, Thiomorpholin-, 1-Oxo-thiomorpholin-, 1,1-Dioxo-thiomorpholin- oder Hexamethylenimin-Rest bilden,
3.7 -O-($C_1$-$C_6$)-Alkyl, das einfach, zweifach oder dreifach substituiertist durch

3.7.1 -OH,
3.7.2 -SH,
3.7.3 =O,
3.7.4 -COOH,
3.8 -COOH,
3.9 -C(O)-O-$R^8$, worin $R^8$ ($C_1$-$C_6$)-Alkyl bedeutet,
3.10 Phenyl,
3.11 Phenyl, das einfach, zweifach oder dreifach substituiert ist durch

3.11.1 -O-($C_1$-$C_4$)-Alkyl,
3.11.2 -O-Phenyl,
3.11.3 ($C_1$-$C_4$)-Alkyl,

3.11.4 $-NO_2$,
3.11.5 Halogen,
3.11.6 $-C(R^9)(R^{10})R^{11}$, worin $R^9$, $R^{10}$ und $R^{11}$ unabhängig voneinander Wasserstoff oder Halogen bedeuten,
3.12 einen Rest eines Heterocyclus aus der Gruppe Morpholin, Piperazin, Imidazol, Piperidin, Pyrrolidin, Pyridin, Thiomorpholin, 1-Oxo-thiomorpholin, 1,1-Dioxo-thiomorpholin, Hexamethylenimin, Pyrrol, Pyrazol, Pyridazin, Pyrazin, Pyrimidin, Indolizin, Indol, Indazol, Purin, Chinoxalin, Furan, Chinazolin, Cinnolin, Pteridin, Oxazol, Isoxazol, Thiazol, Isothiazol, Furazan, Indolin, Pyrazolin, Thiophen, Xanthin, Imidazolin und Pyran,
3.13 einen Rest eines in 3.12 beschriebenen Heterocyclus, der einfach, zweifach, dreifach oder vierfach substituiert ist durch

3.13.1 $(C_1-C_4)$-Alkyl,
3.13.2 =O,
3.13.3 Halogen,
3.13.4 $-O-(C_1-C_4)$-Alkyl,
3.13.5 $-NO_2$,

4. $(C_3-C_7)$-Cycloalkyl, das unsubstituiert ist oder substituiert ist durch

4.1 $(C_1-C_4)$-Alkyl,
4.2 -OH,
4.3 $-O-(C_1-C_4)$-Alkyl,
4.4 $-NH_2$,

$R^3$ für Wasserstoff oder Methoxy steht,
Ar für Phenyl steht, das einfach, zweifach oder dreifach substituiert ist durch

1. Halogen,
2. $-NO_2$,
3. $-O-(C_1-C_6)$-Alkyl,
4. $(C_1-C_6)$-Alkyl, das unsubstituiert ist oder einfach, zweifach oder dreifach substituiert ist durch Halogen,
5. -CN,
6. $-C(O)-N(R^{12})R^{13}$, worin $R^{12}$ und $R^{13}$ gleich oder verschieden sind und unabhängig voneinander Wasserstoff oder $(C_1-C_6)$-Alkyl bedeuten.

**2.** Verbindung der Formel I gemäß Anspruch 1 und/oder stereoisomere Formen der Verbindung der Formel I und/oder Mischungen solcher Formen in allen Verhältnissen und/oder physiologisch verträgliche Salze der Verbindung der Formel I, worin
einer der Reste $R^1$ und $R^2$ für Cyclohexyl steht, das unsubstituiert ist oder substituiert ist durch -OH,
und der andere der Reste $R^1$ und $R^2$ steht für

1. Wasserstoff,
2. $(C_1-C_3)$-Alkyl,
3. $(C_1-C_3)$-Alkyl, das einfach, zweifach oder dreifach substituiert ist durch

3.1 -OH,
3.2 $-O-(C_1-C_3)$-Alkyl,
3.3 $-N(R^6)R^7$, worin $R^6$ und $R^7$ gleich oder verschieden sind und unabhängig voneinander Wasserstoff oder $(C_1-C_3)$-Alkyl bedeuten,
3.4 $-C(O)-N(R^6)R^7$, worin $R^6$ und $R^7$ gleich oder verschieden sind und unabhängig voneinander Wasserstoff oder $(C_1-C_4)$-Alkyl bedeuten, oder $R^6$ und $R^7$ zusammen mit dem N-Atom, an das sie gebunden sind, einen Morpholin-, Piperazin-, Imidazol-, Piperidin-, Pyrrolidin-, Thiomorpholin-, 1-Oxo-thiomorpholin-, 1,1-Dioxo-thiomorpholin- oder Hexamethylenimin-Rest bilden,
3.5 $-O-(C_1-C_6)$-Alkyl, das einfach substituiert ist durch -OH,
3.6 -COOH,
3.7 $-C(O)-O-R^8$, worin $R^8$ $(C_1-C_4)$-Alkyl bedeutet,
3.8 Phenyl,

3.9 Phenyl, das einfach, zweifach oder dreifach substituiert ist durch

3.9.1 -O-($C_1$-$C_4$)-Alkyl,
3.9.2 -O-Phenyl,
3.9.3 ($C_1$-$C_4$)-Alkyl,
3.9.4 -$NO_2$,

3.10 einen Rest eines Heterocyclus aus der Gruppe Morpholin, Piperazin, Imidazol, Piperidin, Pyrrolidin, Pyridin, Thiomorpholin, 1-Oxo-thiomorpholin, 1,1-Dioxo-thiomorpholin, Hexamethylenimin, Pyrrol, Pyrazol, Purin und Pyrimidin,
3.11 einen Rest eines in 3.10 beschriebenen Heterocyclus, der einfach, zweifach, dreifach oder vierfach substituiert ist durch

3.11.1 ($C_1$-$C_4$)-Alkyl,
3.11.2 =O,

4. ($C_5$-$C_6$)-Cycloalkyl, das unsubstituiert ist oder substituiert ist durch

4.1 ($C_1$-$C_4$)-Alkyl,
4.2 -OH,
4.3 -O-($C_1$-$C_4$)-Alkyl,
4.4 -$NH_2$,

$R^3$ für Wasserstoff oder Methoxy steht,
Ar für Phenyl steht, das einfach, zweifach oder dreifach substituiert ist durch

1. Halogen,
2. -$NO_2$,
3. -O-($C_1$-$C_3$)-Alkyl,
4. ($C_1$-$C_2$)-Alkyl, das unsubstituiert ist oder einfach, zweifach oder dreifach substituiert ist durch Halogen,
5. -C(O)-$NH_2$.

**3.** Verbindung der Formel I gemäß Anspruch 1 und/oder Anspruch 2 und/oder stereoisomere Formen der Verbindung der Formel I und/oder Mischungen solcher Formen in allen Verhältnissen und/oder physiologisch verträgliche Salze der Verbindung der Formel I, worin
einer der Reste $R^1$ und $R^2$ für Cyclohexyl steht, das unsubstituiert ist oder substituiert ist durch -OH,
und der andere der Reste $R^1$ und $R^2$ steht für

1. Wasserstoff,
2. ($C_1$-$C_3$)-Alkyl,
3. ($C_1$-$C_3$)-Alkyl, das einfach oder zweifach substituiert ist durch

3.1 -OH,
3.2 -O-$CH_3$,
3.3 -N($R^6$)$R^7$, worin $R^6$ und $R^7$ gleich oder verschieden sind und unabhängig voneinander Wasserstoff oder ($C_1$-$C_3$)-Alkyl bedeuten,
3.4 -O-($C_1$-$C_2$)-Alkyl, das einfach oder zweifach substituiert ist durch -OH,
3.5 einen Rest eines Heterocyclus aus der Gruppe Morpholin und Pyridin,
4. ($C_5$-$C_6$)-Cycloalkyl, das unsubstituiert ist oder substituiert ist durch

4.1 ($C_1$-$C_4$)-Alkyl,
4.2 -OH,
4.3 -O-($C_1$-$C_4$)-Alkyl,
4.4 -$NH_2$,

$R^3$ für Wasserstoff oder Methoxy steht,
Ar für Phenyl steht, das einfach, zweifach oder dreifach substituiert ist durch

1. Halogen,
2. $-NO_2$,
3. $-O-(C_1-C_3)$-Alkyl,
4. $(C_1-C_2)$-Alkyl, das unsubstituiert ist oder einfach, zweifach oder dreifach substituiert ist durch Halogen,
5. $-C(O)-NH_2$.

**4.** Verbindung der Formel I gemäß einem oder mehreren der Ansprüche 1 bis 3 und/oder stereoisomere Formen der Verbindung der Formel I und/oder Mischungen solcher Formen in allen Verhältnissen und/oder physiologisch verträgliche Salze der Verbindung der Formel I, worin einer der Reste $R^1$ und $R^2$ für unsubstituiertes oder -OH substituiertes Cyclohexyl steht und der andere der Reste $R^1$ und $R^2$ für Wasserstoff steht.

**5.** Verbindung der Formel I gemäß einem oder mehreren der Ansprüche 1 bis 4 und/oder stereoisomere Formen der Verbindung der Formel I und/oder Mischungen solcher Formen in allen Verhältnissen und/oder physiologisch verträgliche Salze der Verbindung der Formel I, worin einer der Reste $R^1$ und $R^2$ für durch Hydroxy substituiertes Cyclohexyl steht und der andere der Reste $R^1$ und $R^2$ für Wasserstoff steht.

**6.** Verbindung der Formel I gemäß einem oder mehreren der Ansprüche 1, 4 und 5 und/oder stereoisomere Formen der Verbindung der Formel I und/oder Mischungen solcher Formen in allen Verhältnissen und/oder physiologisch verträgliche Salze der Verbindung der Formel I, worin
einer der Reste $R^1$ und $R^2$ für Hydroxycyclohexyl steht und der andere der Reste $R^1$ und $R^2$ für Wasserstoff steht,

$R^3$ für Wasserstoff oder Methoxy steht,
Ar für Phenyl steht, das einfach, zweifach oder dreifach substituiert ist durch

1. Halogen,
2. $-NO_2$,
3. $-O-(C_1-C_6)$-Alkyl,
4. $(C_1-C_6)$-Alkyl, das unsubstituiert ist oder einfach, zweifach oder dreifach substituiert ist durch Halogen,
5. -CN,
6. $-C(O)-N(R^{12})R^{13}$, worin $R^{12}$ und $R^{13}$ gleich oder verschieden sind und unabhängig voneinander Wasserstoff oder $(C_1-C_6)$-Alkyl bedeuten.

**7.** Verbindung der Formel I gemäß einem oder mehreren der Ansprüche 1 bis 6 und/oder physiologisch verträgliche Salze der Verbindung der Formel I, **dadurch gekennzeichnet, daß** die Verbindung der Formel 1 das 2-(4-Chlorphenyl)-4-(trans-4-hydroxy-cyclohexylamino)-6,7-dimethoxychinazolin,
2-(4-Chlorphenyl)-4-(trans-4-hydroxy-cyclohexylamino)-6,7,8-trimethoxychinazolin, oder
2-(4-Methylphenyl)-4-(trans-4-hydroxy-cyclohexylamino)-6,7-dimethoxychinazolin
ist.

**8.** Verfahren zur Herstellung einer Verbindung der Formel I gemäß einem oder mehreren der Ansprüche 1 bis 7, **dadurch gekennzeichnet, daß** man eine Verbindung der Formel VI

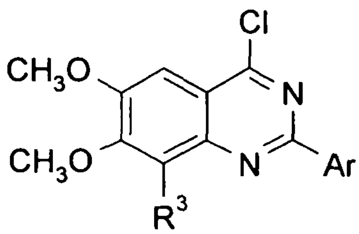

VI

mit einem Amin der Formel $HN(R^1)R^2$ umsetzt, wobei $R^1$, $R^2$, $R^3$ und Ar die in den Ansprüchen 1 bis 7 angegebenen Bedeutungen haben.

**9.** Verbindung der Formel I gemäß einem oder mehreren der Ansprüche 1 bis 7 und/oder ihre physiologisch verträglichen Salze zur Anwendung als Arzneimittel.

**10.** Pharmazeutisches Präparat, **gekennzeichnet durch** einen wirksamen Gehalt an mindestens einer Verbindung der Formel I gemäß einem oder mehreren der Ansprüche 1 bis 7 und/oder eines physiologisch verträglichen Salzes davon zusammen mit einem physiologisch verträglichen Träger.

**11.** Verwendung einer Verbindung der Formel I gemäß einem oder mehreren der Ansprüche 1 bis 7 und/oder eines physiologisch verträglichen Salzes davon zur Herstellung von Arzneimitteln zur Therapie oder Prophylaxe von Erkrankungen, die mit einem niedrigen cGMP-Spiegel in Verbindung stehen.

**12.** Verwendung einer Verbindung der Formel I gemäß einem oder mehreren der Ansprüche 1 bis 7 und/oder eines physiologisch verträglichen Salzes davon zur Herstellung von Arzneimitteln zur Therapie oder Prophylaxe von Herz-Kreislauf-Erkrankungen, endothelialer Dysfunktion, diastolischer Dysfunktion, Atherosklerose, Bluthochdruck, stabiler oder instabiler Angina pectoris, Thrombosen, Restenosen, Myocardinfarkt, Schlaganfällen, Herzinsuffizienz, Pulmonalhypertonie, erektiler Dysfunktion, Asthma bronchiale, chronischer Niereninsuffizienz oder Diabetes oder zur Therapie der Leberzirrhose oder zur Verbesserung einer eingeschränkten Lernfähigkeit oder Gedächtnisleistung.

**Claims**

**1.** A compound of the formula I

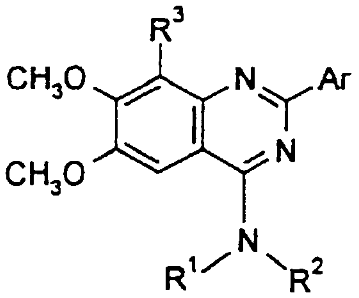

I

and/or stereoisomeric forms of the compound of the formula I and/or mixtures of such forms in all ratios and/or physiologically tolerable salts of the compound of the formula I, in which
one of the radicals $R^1$ and $R^2$ is cyclohexyl which is unsubstituted or substituted by -OH,
and the other one of the radicals $R^1$ and $R^2$ is

1. hydrogen,
2. $(C_1-C_5)$-alkyl,
3. $(C_1-C_5)$-alkyl, which is mono-, di- or tri-substituted by

    3.1 -OH,
    3.2 -O-$(C_1-C_6)$-alkyl,
    3.3 -SH,
    3.4 -$SR^4$, in which $R^4$ is $(C_1-C_6)$ -alkyl,
    3.5 -N $(R^6)$ $R^7$, in which $R^6$ and $R^7$ are identical or different and independently of one another are hydrogen or $(C_1-C_6)$-alkyl,
    3.6 -C(O)-N$(R^6)R^7$, in which $R^6$ and $R^7$ are identical or different and independently of one another are hydrogen or $(C_1-C_6)$ -alkyl, or $R^6$ and $R^7$, together with the N atom to which they are bonded, form a morpholine, piperazine, imidazole, piperidine, pyrrolidine, thiomorpholine, 1-oxothiomorpholine, 1,1-dioxothiomorpholine or hexamethyleneimine radical,
    3.7 -O-$(C_1-C_6)$-alkyl, which is mono-, di- or trisubstituted by

        3.7.1 -OH,
        3.7.2 -SH,
        3.7.3 =O,
        3.7.4 -COOH,
        3.8 -COOH,

3.9 -C(O)-O-$R^8$, in which $R^8$ is ($C_1$-$C_6$) -alkyl,
3.10 phenyl,
3.11 phenyl, which is mono-, di- or trisubstituted by

3.11.1 -O-($C_1$-$C_4$) -alkyl,
3.11.2 -O-phenyl,
3.11.3 ($C_1$-$C_4$)-alkyl,
3.11.4 -$NO_2$,
3.11.5 halogen,
3.11.6 -C ($R^9$) ($R^{10}$)$R^{11}$, in which $R^9$, $R^{10}$ and $R^{11}$ independently of one another are hydrogen or halogen,

3.13

3.12 a radical of a heterocycle from the group consisting of morpholine, piperazine, imidazole, piperidine, pyrrolidine, pyridine, thiomorpholine, 1-oxothiomorpholine, 1,1-dioxothiomorpholine, hexamethyleneimine, pyrrole, pyrazole, pyridazine, pyrazine, pyrimidine, indolizine, indole, indazole, purine, quinoxaline, furan, quinazoline, cinnoline, pteridine, oxazole, isoxazole, thiazole, isothiazole, furazan, indoline, pyrazoline, thiophene, xanthine, imidazoline and pyran,
3.13 a radical of a heterocycle described in 3.12, which is mono-, di-, tri- or tetrasubstituted by

3.13.1 ($C_1$-$C_4$)-alkyl,
3.13.2 =O,
3.13.3 halogen,
3.13.4 -O- ($C_1$-$C_4$) -alkyl,
3.13.5 -$NO_2$,

4. ($C_3$-$C_7$)-cycloalkyl, which is unsubstituted or substituted by

4.1 ($C_1$-$C_4$)-alkyl,
4.2 -OH,
4.3 -O- ($C_1$-$C_4$) -alkyl,
4.4 -$NH_2$,

$R^3$ is hydrogen or methoxy,
Ar is phenyl, which is mono-, di- or trisubstituted by

1. halogen,
2. -$NO_2$,
3. -O- ($C_1$-$C_6$) -alkyl,
4. ($C_1$-$C_6$)-alkyl, which is unsubstituted or is mono-, di- or trisubstituted by halogen,
5. -CN,
6. -C(O)-N($R^{12}$)$R^{13}$, in which $R^{12}$ and $R^{13}$ are identical or different and independently of one another are hydrogen or ($C_1$-$C_6$)-alkyl.

**2.** A compound of the formula I as claimed in claim 1 and/or stereoisomeric forms of the compound of the formula I and/or mixtures of such forms in all ratios and/or physiologically tolerable salts of the compound of the formula I, in which
one of the radicals $R^1$ and $R^2$ is cyclohexyl which is unsubstituted or substituted by -OH,
and the other one of the radicals $R^1$ and $R^2$ is

1. hydrogen,
2. ($C_1$-$C_3$) -alkyl,
3. ($C_1$-$C_3$) -alkyl, which is mono-, di- or trisubstituted by

3.1 -OH,
3.2 -O-($C_1$-$C_3$)-alkyl,
3.3 -N ($R^6$) $R^7$, in which $R^6$ and $R^7$ are identical or different and independently of one another are hydrogen

or $(C_1-C_3)$ -alkyl,
3.4 -C(O)-N($R^6$)$R^7$, in which $R^6$ and $R^7$ are identical or different and independently of one another are hydrogen or $(C_1-C_4)$-alkyl, or $R^6$ and $R^7$, together with the N atom to which they are bonded, are a morpholine, piperazine, imidazole, piperidine, pyrrolidine, thiomorpholine, 1-oxothiomorpholine, 1,1-dioxothiomorpholine or hexamethyleneimine radical,
3.5 -O-$(C_1-C_6)$-alkyl, which is monosubstituted by -OH,
3.6 -COOH,
3.7 -C(O)-O-$R^8$, in which $R^8$ is $(C_1-C_4)$ -alkyl,
3.8 phenyl,
3.9 phenyl which is mono-, di- or trisubstituted by

3. 9. 1 -O-$(C_1-C_4)$-alkyl,
3.9.2 -0-phenyl,
3.9.3 $(C_1-C_4)$-alkyl,
3.9.4 -$NO_2$,

3.10 a radical of a heterocycle from the group consisting of morpholine, piperazine, imidazole, piperidine, pyrrolidine, pyridine, thiomorpholine, 1-oxothiomorpholine, 1,1-dioxothiomorpholine, hexamethyleneimine, pyrrole, pyrazole, purine and pyrimidine,
3.11 a radical of a heterocycle described in 3.10, which is mono-, di-, tri- or tetrasubstituted by

3.11.1 $(C_1-C_4)$ -alkyl,
3.11.2 =0,

4. $(C_5-C_6)$-cycloalkyl which is unsubstituted or is substituted by

4.1 $(C_1-C_4)$-alkyl,
4.2 -OH,
4.3 -O-$(C_1-C_4)$-alkyl,
4.4 -$NH_2$,

$R^3$ is hydrogen or methoxy,
Ar is phenyl, which is mono-, di- or trisubstituted by

1. halogen,
2. -$NO_2$,
3. -O-$(C_1-C_3)$ -alkyl,
4. $(C_1-C_2)$ -alkyl, which is unsubstituted or is mono-, di- or trisubstituted by halogen,
5. -C(O)-$NH_2$.

**3.** A compound of the formula I as claimed in claim 1 and/or claim 2 and/or stereoisomeric forms of the compound of the formula I and/or mixtures of such forms in all ratios and/or physiologically tolerable salts of the compound of the formula I, in which one of the radicals $R^1$ and $R^2$ is cyclohexyl which is unsubstituted or substituted by -OH,
and the other one of the radicals $R^1$ and $R^2$ is

1. hydrogen,
2. $(C_1-C_3)$ -alkyl,
3. $(C_1-C_3)$-alkyl, which is mono- or disubstituted by

3.1 -OH,
3.2 -O-$CH_3$,
3.3 -N($R^6$) $R^7$, in which $R^6$ and $R^7$ are identical or different and independently of one another are hydrogen or $(C_1-C_3)$ -alkyl,
3.4 -O-$(C_1-C_2)$-alkyl, which is mono- or disubstituted by -OH,
3.5 a radical of a heterocycle from the group consisting of morpholine and pyridine,

4. $(C_5-C_6)$-cycloalkyl, which is unsubstituted or is substituted by

4.1 $(C_1-C_4)$-alkyl,
4.2 -OH,
4.3 -O- $(C_1-C_4)$ -alkyl,
4.4 $-NH_2$,

$R^3$ is hydrogen or methoxy,
Ar is phenyl, which is mono-, di- or trisubstituted by

1. halogen,
2. $-NO_2$,
3. $-O-(C_1-C_3)$-alkyl,
4. $(C_1-C_2)$-alkyl, which is unsubstituted or is mono-, di- or trisubstituted by halogen,
5. $-C(O)-NH_2$.

**4.** A compound of the formula I as claimed in one or more of claims 1 to 3 and/or stereoisomeric forms of the compound of the formula I and/or mixtures of such forms in all ratios and/or physiologically tolerable salts of the compound of the formula I, in which one of the radicals $R^1$ and $R^2$ is unsubstituted or -OH-substituted cyclohexyl and the other one of the radicals $R^1$ and $R^2$ is hydrogen.

**5.** A compound of the formula I as claimed in one or more of claims 1 to 4 and/or stereoisomeric forms of the compound of the formula I and/or mixtures of such forms in all ratios and/or physiologically tolerable salts of the compound of the formula I, in which one of the radicals $R^1$ and $R^2$ is hydroxyl-substituted cyclohexyl and the other one of the radicals $R^1$ and $R^2$ is hydrogen.

**6.** A compound of the formula I as claimed in one or more of claims 1, 4 and 5 and/or stereoisomeric forms of the compound of the formula I and/or mixtures of such forms in all ratios and/or physiologically tolerable salts of the compound of the formula I, in which
one of the radicals $R^1$ and $R^2$ is hydroxycyclohexyl and the other one of the radicals $R^1$ and $R^2$ is hydrogen,

$R^3$ is hydrogen or methoxy,
Ar is phenyl, which is mono-, di- or trisubstituted by

1. halogen,
2. $-NO_2$,
3. -O- $(C_1-C_6)$ -alkyl,
4. $(C_1-C_6)$-alkyl, which is unsubstituted or is mono-, di- or trisubstituted by halogen,
5. -CN,
6. -C (O) -N $(R^{12})$ $R^{13}$, in which $R^{12}$ and $R^{13}$ are identical or different and independently of one another are hydrogen or $(C_1-C_6)$-alkyl.

**7.** A compound of the formula I as claimed in one or more of claims 1 to 6 and/or physiologically tolerable salts of the compound of the formula I, wherein the compound of the formula I is 2-(4-chlorophenyl)-4-(trans-4-hydroxycyclohexylamino)-6,7-dimethoxyquinazoline, 2-(4-chlorophenyl)-4-(trans-4-hydroxycyclohexylamino)-6,7,8-trimethoxyquinazoline, or 2-(4-methylphenyl)-4-(trans-4-hydroxycyclohexylamino)-6,7-dimethoxyquinazoline.

**8.** A process for the preparation of a compound of the formula I as claimed in one or more of claims 1 to 7, which comprises reacting a compound of the formula VI

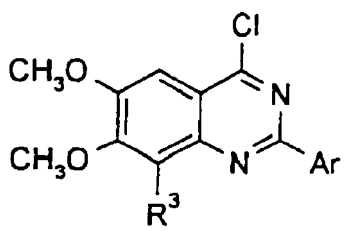

VI

with an amine of the formula HN ($R^1$) $R^2$, where $R^1$, $R^2$, $R^3$ and Ar have the meanings indicated in claims 1 to 7.

**9.** A compound of the formula I as claimed in one or more of claims 1 to 7 and/or its physiologically tolerable salts for use as a pharmaceutical.

**10.** A pharmaceutical preparation which comprises an efficacious amount of at least one compound of the formula I as claimed in one or more of claims 1 to 7 and/or a physiologically tolerable salt thereof together with a physiologically tolerable carrier.

**11.** The use of a compound of the formula I as claimed in one or more of claims 1 to 7 and/or of a physiologically tolerable salt thereof for the production of pharmaceuticals for the therapy or prophylaxis of diseases which are associated with a low cGMP level.

**12.** The use of a compound of the formula I as claimed in one or more of claims 1 to 7 and/or of a physiologically tolerable salt thereof for the production of pharmaceuticals for the therapy or prophylaxis of cardiovascular diseases, endothelial dysfunction, diastolic dysfunction, atherosclerosis, high blood pressure, stable or unstable angina pectoris, thromboses, restenoses, myocardial infarcts, strokes, cardiac insufficiency, pulmonary hypertension, erectile dysfunction, bronchial asthma, chronic renal insufficiency or diabetes or for the therapy of cirrhosis of the liver or for improving restricted learning ability or memory power.

## Revendications

**1.** Composé de formule I

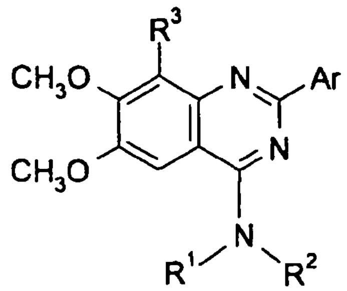

I

et/ou formes stéréo-isomères du composé de formule I et/ou mélanges de ces formes dans tous les rapports et/ou sels physiologiquement acceptables du composé de formule I, dans laquelle
un des radicaux $R^1$ et $R^2$ représente cyclohexyle, qui est non substitué ou substitué par -OH,
et l'autre des radicaux $R^1$ et $R^2$ représente

1. hydrogène,
2. alkyle en $C_1$ à $C_5$,
3. alkyle en $C_1$ à $C_5$ qui est monosubstitué, disubstitué ou trisubstitué par

    3.1 -OH,
    3.2 -O-alkyle en $C_1$ à $C_6$,
    3.3 -SH,
    3.4 -$SR^4$, où $R^4$ signifie alkyle en $C_1$ à $C_6$,
    3.5 -N($R^6$) $R^7$, où $R^6$ et $R^7$ sont identiques ou différents et représentent, indépendamment l'un de l'autre, hydrogène ou alkyle en $C_1$ à $C_6$,
    3.6 -C(O)-N($R^6$)$R^7$, où $R^6$ et $R^7$ sont identiques ou différents et signifient, indépendamment l'un de l'autre, hydrogène ou alkyle en $C_1$ à $C_6$, ou $R^6$ et $R^7$ forment ensemble avec l'atome de N auquel ils sont liés un radical morpholine, pipérazine, imidazole, pipéridine, pyrrolidine, thiomorpholine, 1-oxo-thiomorpholine, 1,1-dioxo-thiomorpholine ou hexaméthylèneimine,
    3.7 -O-alkyle en $C_1$ à $C_6$ qui est monosubstitué, disubstitué ou trisubstitué par

3.7.1 -OH,
3.7.2 -SH,
3.7.3 =O,
3.7.4 -COOH,
3.8 -COOH,
3.9 -C(O)-O-$R^8$, où $R^8$ signifie alkyle en $C_1$ à $C_6$,
3.10 phényle,
3.11 phényle, qui est monosubstitué, disubstitué ou trisubstitué par

3.11.1 -O-alkyle en $C_1$ à $C_4$,
3.11.2 -O-phényle,
3.11.3 alkyle en $C_1$ à $C_4$,
3.11.4 -$NO_2$,
3.11.5 halogène,
3.11.6 -C($R^9$) ($R^{10}$)$R^{11}$, où $R^9$, $R^{10}$ et $R^{11}$ signifient, indépendamment l'un de l'autre, hydrogène ou halogène,

3.12 un radical d'un hétérocycle du groupe formé par la morpholine, la pipérazine, l'imidazole, la pipéridine, la pyrrolidine, la pyridine, la thiomorpholine, la 1-oxo-thiomorpholine, la 1,1-dioxo-thiomorpholine, l'hexaméthylèneimine, le pyrrole, le pyrazole, la pyridazine, la pyrazine, la pyrimidine, l'indolizine, l'indole, l'indazole, la purine, la quinoxaline, le furanne, la quinazoline, la cinnoline, la ptéridine, l'oxazole, l'isoxazole, le thiazole, l'isothiazole, le furazane, l'indoline, la pyrazoline, le thiophène, la xanthine, l'imidazoline et le pyranne,
3.13 un radical d'un hétérocycle décrit au point 3.12 qui est monosubstitué, disubstitué, trisubstitué ou tétrasubstitué par

3.13.1 alkyle en $C_1$ à $C_4$,
3.13.2 =0,
3.13.3 halogène,
3.13.4 -O-alkyle en $C_1$ à $C_4$,
3.13.5 -$NO_2$,

4.ycloalkyle en $C_3$ à $C_7$, qui est non substitué ou substitué par

4.1 alkyle en $C_1$ à $C_4$,
4.2 -OH,
4.3 -0-alkyle en $C_1$ à $C_4$,
4.4 -$NH_2$,

$R^3$ représente hydrogène ou méthoxy,
Ar représente phényle qui est monosubstitué, disubstitué ou trisubstitué par

1. halogène
2. -$NO_2$,
3. -O-alkyle en $C_1$ à $C_6$,
4. alkyle en $C_1$ à $C_6$, qui est non substitué ou monosubstitué, disubstitué ou trisubstitué par halogène,
5. -CN,
6. -C (O) -N ($R^{12}$) $R^{13}$, où $R^{12}$ et $R^{13}$ sont identiques ou différents et représentent, indépendamment l'un de l'autre, hydrogène ou alkyle en $C_1$ à $C_6$.

**2.** Composé de formule I selon la revendication 1 et/ou formes stéréo-isomères du composé de formule I et/ou mélanges de ces formes dans tous les rapports et/ou sels physiologiquement acceptables du composé de formule I, dans laquelle
un des radicaux $R^1$ et $R^2$ représente cyclohexyle, qui est non substitué ou substitué par -OH,
et l'autre des radicaux $R^1$ et $R^2$ représente

1. hydrogène,

2. alkyle en $C_1$ à $C_3$,
3. alkyle en $C_1$ à $C_3$ qui est monosubstitué, disubstitué ou trisubstitué par

3.1 -OH,
3.2 -O-alkyle en $C_1$ à $C_3$,
3. 3 -N ($R^6$) $R^7$, où $R^6$ et $R^7$ sont identiques ou différents et représentent, indépendamment l'un de l'autre, hydrogène ou alkyle en $C_1$ à $C_3$,
3.4 -C (O) -N ($R^6$) $R^7$, où $R^6$ et $R^7$ sont identiques ou différents et signifient, indépendamment l'un de l'autre, hydrogène ou alkyle en $C_1$ à $C_4$, ou $R^6$ et $R^7$ forment ensemble avec l'atome de N auquel ils sont liés un radical morpholine, pipérazine, imidazole, pipéridine, pyrrolidine, thiomorpholine, 1-oxo-thiomorpholine, 1,1-dioxo-thiomorpholine ou hexaméthylèneimine,
3.5 -O-alkyle en $C_1$ à $C_6$, qui est monosubstitué par -OH,
3.6 -COOH,
3.7 -C(O)-O-$R^8$, où $R^8$ signifie alkyle en $C_1$ à $C_4$,
3.8 phényle,
3.9 phényle, qui est monosubstitué, disubstitué ou trisubstitué par

3.9.1 -O-alkyle en $C_1$ à $C_4$,
3.9.2 -O-phényle,
3.9.3 alkyle en $C_1$ à $C_4$,
3.9.4 -$NO_2$,

3.10 un radical d'un hétérocycle du groupe formé par la morpholine, la pipérazine, l'imidazole, la pipéridine, la pyrrolidine, la pyridine, la thiomorpholine, la 1-oxo-thiomorpholine, la 1,1-dioxo-thiomorpholine, l'hexaméthylèneimine, le pyrrole, le pyrazole, la purine et la pyrimidine,
3.11 un radical d'un hétérocycle décrit au point 3.10 qui est monosubstitué, disubstitué, trisubstitué ou tétrasubstitué par

3.11.1 alkyle en $C_1$ à $C_4$,
3.11.2 =O,

4. cycloalkyle en $C_5$ à $C_6$, qui est non substitué ou substitué par

4.1 alkyle en $C_1$ à $C_4$,
4.2 -OH,
4.3 -O-alkyle en $C_1$ à $C_4$,
4.4 -$NH_2$,

$R^3$ représente hydrogène ou méthoxy,
Ar représente phényle qui est monosubstitué, disubstitué ou trisubstitué par

1. halogène,
2. -$NO_2$,
3. -O-alkyle en $C_1$ à $C_3$,
4. alkyle en $C_1$ à $C_2$, qui est non substitué ou monosubstitué, disubstitué ou trisubstitué par halogène,
5. -C(O)-$NH_2$.

**3.** Composé de formule I selon la revendication 1 et/ou la revendication 2 et/ou formes stéréo-isomères du composé de formule I et/ou mélanges de ces formes dans tous les rapports et/ou sels physiologiquement acceptables du composé de formule I, dans laquelle
un des radicaux $R^1$ et $R^2$ représente cyclohexyle, qui est non substitué ou substitué par -OH,
et l'autre des radicaux $R^1$ et $R^2$ représente

1. hydrogène,
2. alkyle en $C_1$ à $C_3$,
3. alkyle en $C_1$ à $C_3$ qui est monosubstitué ou disubstitué par

3.1 -OH,

3.2 $-O-CH_3$,
3.3 -N ($R^6$) $R^7$, où $R^6$ et $R^7$ sont identiques ou différents et représentent, indépendamment l'un de l'autre, hydrogène ou alkyle en $C_1$ à $C_3$,
3.4 -O-alkyle en $C_1$ à $C_2$ qui est monosubstitué ou disubstitué par -OH,
3.5 un radical d'un hétérocycle du groupe formé par la morpholine et la pyridine,

4. cycloalkyle en $C_5$ à $C_6$, qui est non substitué ou substitué par

4.1 alkyle en $C_1$ à $C_4$,
4.2 -OH,
4.3 -O-alkyle en $C_1$ à $C_4$,
4.4 $-NH_2$,

$R^3$ représente hydrogène ou méthoxy,
Ar représente phényle qui est monosubstitué, disubstitué ou trisubstitué par

1. halogène,
2. $-NO_2$,
3. -O-alkyle en $C_1$ à $C_3$,
4. alkyle en $C_1$ à $C_2$, qui est non substitué ou monosubstitué, disubstitué ou trisubstitué par halogène,
5. $-C(O)-NH_2$.

**4.** Composé de formule I selon l'une ou plusieurs des revendications 1 à 3 et/ou formes stéréo-isomères du composé de formule I et/ou mélanges de ces formes dans tous les rapports et/ou sels physiologiquement acceptables du composé de formule I, dans laquelle un des radicaux $R^1$ et $R^2$ représente cyclohexyle non substitué ou substitué par -OH et l'autre des radicaux $R^1$ et $R^2$ représente hydrogène.

**5.** Composé de formule I selon l'une ou plusieurs des revendications 1 à 4 et/ou formes stéréo-isomères du composé de formule I et/ou mélanges de ces formes dans tous les rapports et/ou sels physiologiquement acceptables du composé de formule I, dans laquelle un des radicaux $R^1$ et $R^2$ représente cyclohexyle substitué par hydroxy et l'autre des radicaux $R^1$ et $R^2$ représente hydrogène.

**6.** Composé de formule I selon l'une ou plusieurs des revendications 1, 4 et 5 et/ou formes stéréo-isomères du composé de formule I et/ou mélanges de ces formes dans tous les rapports et/ou sels physiologiquement acceptables du composé de formule I, dans laquelle
un des radicaux $R^1$ et $R^2$ représente hydroxycyclohexyle et l'autre des radicaux $R^1$ et $R^2$ représente hydrogène,
$R^3$ représente hydrogène ou méthoxy,
Ar représente phényle qui est monosubstitué, disubstitué ou trisubstitué par

1. halogène,
2. $-NO_2$,
3. -O-alkyle en $C_1$ à $C_6$,
4. alkyle en $C_1$ à $C_6$, qui est non substitué ou monosubstitué, disubstitué ou trisubstitué par halogène,
5. -CN,
6. -C(O)-N($R^{12}$)$R^{13}$, où $R^{12}$ et $R^{13}$ sont identiques ou différents et représentent, indépendamment l'un de l'autre, hydrogène ou alkyle en $C_1$ à $C_6$,

**7.** Composé de formule I selon l'une ou plusieurs des revendications 1 à 6 et/ou sels physiologiquement acceptables du composé de formule I, **caractérisé en ce que** le composé de formule I est
la 2-(4-chlorophényl)-4-(trans-4-hydroxy-cyclohexylamino)-6,7-diméthoxyquinazoline,
la 2-(4-chlorophényl)-4-(trans-4-hydroxy-cyclohexylamino)-6,7,8-triméthoxyquinazoline, ou
la 2-(4-méthylphényl)-4-(trans-4-hydroxy-cyclohexylamino)-6,7-diméthoxyquinazoline.

**8.** Procédé pour la préparation d'un composé de formule I selon l'une ou plusieurs des revendications 1 à 7, **caractérisé en ce qu'**on transforme un composé de formule VI

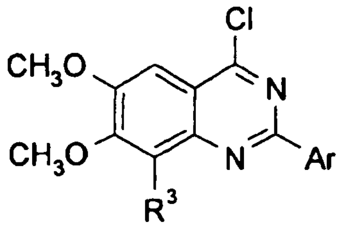

VI

avec une amine de formule HN ($R^1$) $R^2$, où $R^1$, $R^2$, $R^3$ et Ar ont les significations indiquées dans les revendications 1 à 7.

**9.** Composé de formule I selon l'une ou plusieurs des revendications 1 à 7 et/ou ses sels physiologiquement acceptables pour une utilisation comme médicament.

**10.** Préparation pharmaceutique **caractérisée par** une teneur active en au moins un composé de formule I selon l'une ou plusieurs des revendications 1 à 7 et/ou un sel physiologiquement acceptable de celui-ci avec un support physiologiquement acceptable.

**11.** Utilisation d'un composé de formule I selon l'une ou plusieurs des revendications 1 à 7 et/ou d'un sel physiologiquement acceptable de celui-ci pour la préparation de médicaments destinés à la thérapie ou la prophylaxie de maladies associées à un niveau bas de cGMP.

**12.** Utilisation d'un composé de formule I selon l'une ou plusieurs des revendications 1 à 7 et/ou d'un sel physiologiquement acceptable de celui-ci pour la préparation de médicaments destinés à la thérapie ou la prophylaxie de maladies cardiovasculaires, d'un dysfonctionnement endothélial, d'un dysfonctionnement diastolique, de l'athérosclérose, de l'hypertension, de l'angine de poitrine stable ou instable, de thromboses, de resténoses, de l'infarctus du myocarde, d'attaques, de l'insuffisance cardiaque, de l'hypertonie pulmonaire, du dysfonctionnement érectile, de l'asthme bronchique, de l'insuffisance rénale chronique ou du diabète ou pour la thérapie d'une cirrhose du foie ou pour l'amélioration d'une aptitude diminuée à l'apprentissage ou de la perte de mémoire.